# EUROPEAN PATENT SPECIFICATION

(11) **EP 1 842 455 B1**
(45) Date of publication and mention of the grant of the patent: **31.12.2008**
(21) Application number: 06115851.5
(22) Date of filing: 22.06.2006
(51) Int. Cl.: A47C 23/04

(54) **Spring structure having contact-preventing and rigidity-reinforcing function for bed mattress**
Federstruktur mit erhöhter Starrheit und kontaktvermeidender Funktion, für Bettmatratzen
Ressort d'un matelas avec une structure pour éviter contact interne et pour augmenter l'inflexibilité

(30) Priority: 07.04.2006 KR 2006031670
(43) Date of publication of application: 10.10.2007
(73) Proprietor: Ace Bed Co., Ltd., SeongNam-shi, Kyonggi-Do (KR)
(72) Inventor: Ahn, Yoo Soo, Kyonggi-Do (KR)
(74) Representative: Barth, Stephan Manuel

(56) References cited:
- GB-A- 190 902 592
- US-A- 5 575 460

## Description

### BACKGROUND OF THE INVENTION

### Field of the Invention

The present invention relates to a spring structure having a contact-preventing and rigidity-reinforcing function for a bed mattress, and more particularly to such a spring structure having a contact-preventing and rigidity-reinforcing function for a bed mattress in which the structure of a connection end portion for connecting a body spring and an exposure wire spring to each other is improved such that the connection end portion is not in close contact with an uppermost winding and/or a lowermost winding of the body spring and such that when a load is exerted to an exposure wire spring a displacement is prevented from being generated in which the exposure wire spring is leaned to one side, and rigidity of the spring is reinforced so that the exposure wire spring ascends/descends vertically only, thereby easily preventing the lateral displacement and deformation of the spring along with a prevention of a noise due to a frictional contact to prolong the lifespan of the spring.

A spring core for a mattress is known from US 5,575,460. Identically shaped spiral rings are lying diametrically opposite each other. A spring coil part is arranged centered between the spiral rings.

GB 2592 discloses a coiled spring for mattresses. A spiral spring is provided with coils at both its ends.

### Background of the Related Art

In general, a bed mattress is mounted on a bed frame and is used as means adapted to provide a cushion force and buffering force. The bed mattress basically includes a spring assembly, an intermediate member laminatedly attached on the upper and lower surfaces of the spring assembly, an edge former fittingly attached to the circumferential edge of the spring assembly, and a cover member for protecting the surfaces of the intermediate member and the edge former.

Especially, the spring assembly is composed of springs vertically arranged spaced apart from one another at regular intervals over the entire area of the bed mattress, and a helical coil for securely engaging the springs with one another.

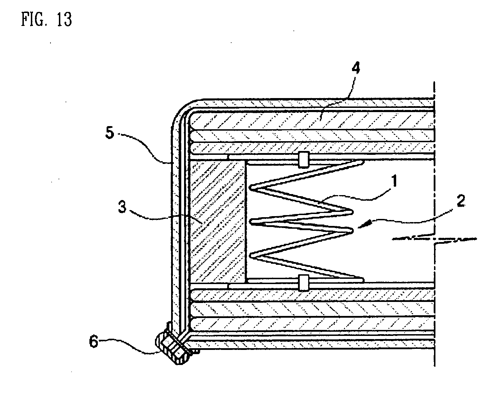

Herein, in order to better understand the present invention, a process for manufacturing a bed mattress will be hereinafter described in brief with reference to FIG. 13.

Referring to FIG. 13, the process for manufacturing the bed mattress includes the following steps of: a step of fabricating a spring assembly 2 including coil springs arranged along row and column directions over the entire area of the bed mattress in such a fashion as to be spaced apart from one another at regular intervals, the coil springs being securely fixed by means of a helical coil; a step of fittingly attaching an edge former as a support means to the circumferential edge of the spring assembly, and then continuously laminating multi-layered cushion means including a felt and a non-woven fabric as an intermediate member 4 on the upper and lower surfaces of the spring assembly 2; a step of covering the upper and lower surfaces and the circumferential edge surface of the intermediate member 4 as well as the outer surface of the edge former 3 with a cover 5, and then hermetically sealing a seam portion of the cover 5 with a sealing means 6.

Therefore, when a user sleeps or takes a rest, a load exerted to the bed mattress is absorbed and buffered by means of a cushion force of the intermediate member and a buffering force of the spring so that he or she can feels convenience and comfort.

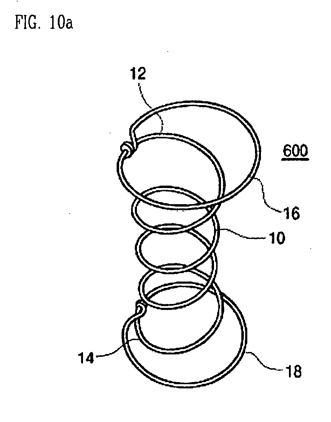

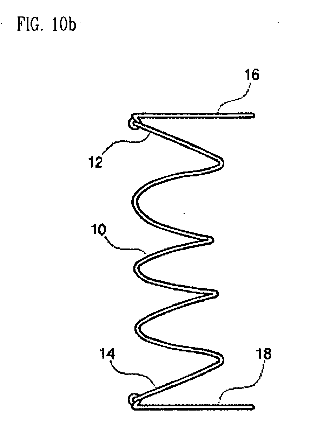

Now, a spring structure for a conventional bed mattress constituting the spring assembly will be described hereinafter with reference to FIGs. 10a and 10b.

FIGs. 10a and 10b illustrate an example of a conventional spring structure.

Referring to FIGs. 10a and 10b, a conventional spring 600 includes a body spring 10 formed in a coil shape whose diameter is gradually increased as it goes toward the top and the bottom from the central portion thereof, an upper end spring 16 wound and extending horizontally at a terminating point of the uppermost winding 12 of the body spring 10, and a lower end spring 18 wound and extending horizontally at a terminating point of the lowermost winding 14 of the body spring 10.

In this case, a distal end of the upper end spring 16 is fixed in such a fashion as to be twisted at the terminating point of the uppermost winding 12 of the body spring 10, and a distal end of the lower end spring 18 is fixed in such a fashion as to be twisted at the terminating point of the lowermost winding 14 of the body spring 10.

However, when the bed mattress is manufactured by employing the conventional spring, there are the following demerits:

Since a load applied to the bed mattress is finally absorbed by the spring, all the loads including a larger load and a smaller load is finally buffered and absorbed by the spring.

In this manner, when different loads are irregularly concentrated on the spring, there is a risk that the deformation of the spring may be progressed rapidly. In addition, adjacent springs come into close contact with each other according to the deformation of the spring to thereby contribute to the generation of a noise.

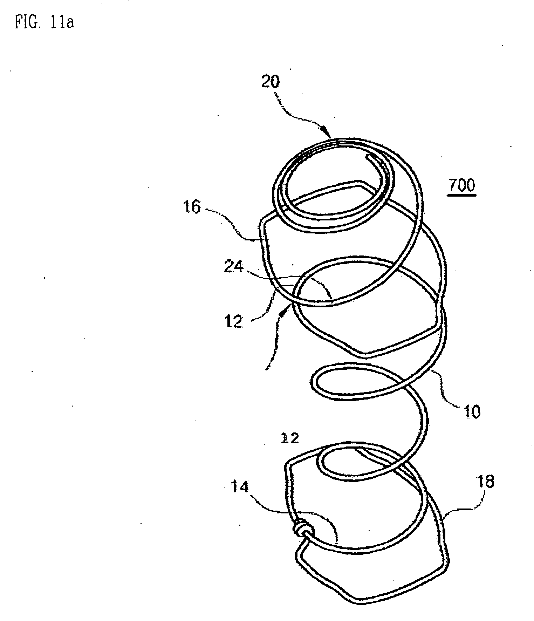

In view of these problems, another type of spring which has a dual buffer structure to decentrally buffer the larger and smaller loads separately has been manufactured, and its shape is shown in FIGs. 11a to 11c.

Referring to FIGs. 11a to 11c, a conventional spring 700 of another type, as shown in FIGs. 10a and 10b, includes a body spring 10 formed in a coil shape whose diameter is gradually increased as it goes toward the top and the bottom from the central portion thereof, an upper end spring 16 wound and extending horizontally at a terminating point of the uppermost winding 12 of the body spring 10, and a lower end spring 18 wound and extending horizontally at a terminating point of the lowermost winding 14 of the body spring 10. The spring 700 is characterized in that a separate exposure wire spring 20 is formed integrally with the upper end spring 16 in such a fashion as to be disposed above the upper end spring 16.

At this time, a distal end of the upper end spring 16 is connected integrally with the exposure wire spring 20, and a distal end of the lower end spring 18 is fixed in such a fashion as to be twisted at the terminating point of the uppermost winding 14 of the body spring 10.

The exposure wire spring 20 has a diameter smaller than that of the body spring 10 and is configured to be wound in a coil shape. The exposure wire spring 20 also has a resilient force causing compressible deformation thereof relatively easily as compared to the body spring 10.

Especially, since a portion 24 (hereinafter, referred to as "connection end portion") extending from the distal end of the upper end spring 16 to the exposure wire spring 20 runs spirally toward a vertical central axis of the overall spring, the connection end portion 24 and the uppermost wiring 12 for the body spring 10 positioned just below the connection end portion intersect each other when viewed from the top.

Accordingly, in the case where a smaller load (just a load applied to the bed mattress when a user twists and turns in his or her bed mattress) is exerted to the bed mattress, the exposure wire spring 20 buffers/absorbs the load impact. On the other hand, in the case where a larger load (a load applied to the bed mattress when the user sits on the bed mattress) is exerted to the bed mattress, the body spring 10 buffers/absorbs the load impact.

As such, the body spring and the exposure wire spring decentrally perform a buffering function thereof separately depending on the magnitude of the load exerted to the bed mattress to thereby provide advantages of preventing the deformation of the spring and prolonging the lifespan of the spring.

But, the conventional spring 700 of another type with the exposure wire spring has the following demerits:

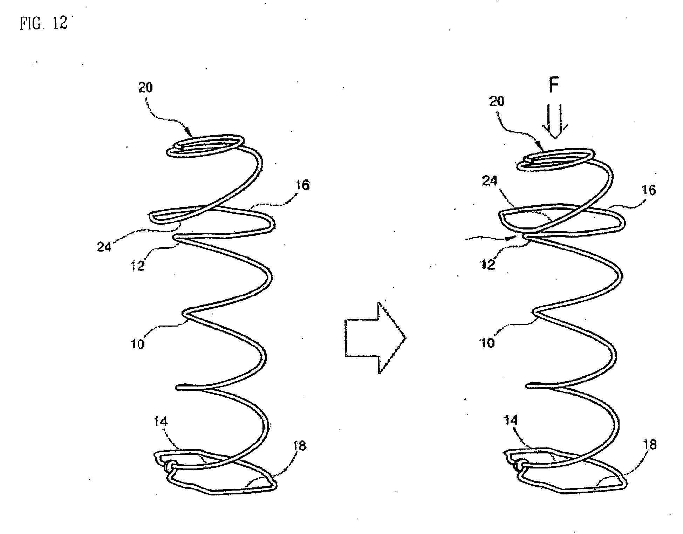

As shown in FIG. 12, when the exposure wire spring 20 is compressed along with the exertion of a load to the bed mattress, and simultaneously the connection end portion 24 for connecting the distal end of the upper end spring 16 and the exposure wire spring 20 to each other is applied with a compression load, the connection end portion 24 and the uppermost wiring 12 for the body spring 10 positioned just below the connection end portion intersect each other when viewed from the top as described above. Thus, the connection end portion 24 comes into close contact with the uppermost wiring 12 while descending, to thereby generate the noise due to the contact therebetween.

Moreover, in the case where a larger load is exerted to the bed mattress, the exposure wire spring 20 is compressed and simultaneously the body spring 10 is also compressed, so that the connection end portion 24 also descends upon the compression of the exposure wire spring 20. At this time, the connection end portion 24 comes into close contact with the uppermost winding 12 of the body spring 10 with a larger impact, to thereby generate a larger contact noise.

As such, the contact noise caused by the spring during the use of the bed mattress acts as a great stress to a user, which may become a critical disadvantage for bed mattress products.

In addition, if the connection end portion repeatedly comes into close with the uppermost winding of the body spring by friction, it will not be long before the spring itself is deformed.

Moreover, since the direction of a load exerted to the bed mattress is not oriented uniformly such as vertically or slantly, there may occur a problem in that the exposure wire spring having a relatively weak rigidity as compared to that of the body spring is easily displaced laterally.

In this manner, when the exposure wire spring 20 is displaced in one direction, there is a problem in that an area of the bed mattress is locally depressed and a uniform cushion sense is not provided over the entire area of the bed mattress.

### SUMMARY OF THE INVENTION

Accordingly, the present invention has been made in view of the aforementioned problems occurring in the prior art, and it is an object of the present invention to provide a spring structure having a contact-preventing and rigidity-reinforcing function for a bed mattress in which the structure of a connection end portion for connecting a body spring and an exposure wire spring to each other is improved in such a fashion as to concurrently achieve a contact-preventing function in which the connection end portion is not in close contact with an uppermost winding and/or a lowermost winding of the body spring as well as a rigidity-reinforcing function in which the exposure wire spring is guided to stably ascend/descend vertically only, but not displaced laterally when a load is exerted to the exposure wire spring.

To accomplish the above object, according to one aspect of the present invention, there is provided a spring structure having a contact-preventing and rigidity-reinforcing function for a bed mattress which comprises a body spring formed in a coil shape, upper and lower end springs wound and extending horizontally at upper and lower portions of the body spring, an exposure wire spring formed integrally with the upper end spring in such a fashion as to be disposed above the upper end spring, and a connection end portion for integrally connecting the upper end spring and the exposure wire spring to each other, wherein a first contact-preventing/rigidity-reinforcing end is formed at the connection end portion in such a fashion as to be bent in a "Z" shape upwardly from a distal end of the upper end spring positioned outwardly from an uppermost winding of the body spring.

According to another aspect of the present invention, there is provided a spring structure having a contact-preventing and rigidity-reinforcing function for a bed mattress which comprises a body spring formed in a coil shape, upper and lower end springs wound and extending horizontally at upper and lower portions of the body spring, an exposure wire spring formed integrally with the upper end spring in such a fashion as to be disposed above the upper end spring, and a connection end portion for integrally connecting the upper end spring and the exposure wire spring to each other, wherein a first contact-preventing/rigidity-reinforcing end is formed at the connection end portion in such a fashion as to be bent in a "Z" shape upwardly from a distal end of the upper end spring positioned outwardly from an uppermost winding of the body spring, and wherein a second contact-preventing/rigidity-reinforcing end is formed at the uppermost winding of the body spring positioned below the first contact-preventing/rigidity-reinforcing end in such a fashion as to be bent in a "Z" shape upwardly from a terminating point of the uppermost winding of the body spring to a starting point of the upper end spring.

According to another aspect of the present invention, there is provided a spring structure having a contact-preventing and rigidity-reinforcing function for a bed mattress which comprises a body spring formed in a coil shape, upper and lower end springs wound and extending horizontally at upper and lower portions of the body spring, an exposure wire spring formed integrally with the upper end spring in such a fashion as to be disposed above the upper end spring, and a connection end portion for integrally connecting the upper end spring and the exposure wire spring to each other, wherein a first contact-preventing/rigidity-reinforcing end is formed at the connection end portion in such a fashion as to be bent in a "Z" shape upwardly from a distal end of the upper end spring positioned outwardly from an uppermost winding of the body spring, and wherein the exposure wire spring is further formed integrally with the lower end spring in such a fashion as to be disposed below the lower end spring, and the first contact-preventing/rigidity-reinforcing end is further formed at a connection end portion extending between the lower end spring and the exposure wire spring.

According to another aspect of the present invention, there is provided a spring structure having a contact-preventing and rigidity-reinforcing function for a bed mattress which comprises a body spring formed in a coil shape, upper and lower end springs wound and extending horizontally at upper and lower portions of the body spring, an exposure wire spring formed integrally with the upper end spring in such a fashion as to be disposed above the upper end spring, and a connection end portion for integrally connecting the upper end spring and the exposure wire spring to each other, wherein a first contact-preventing/rigidity-reinforcing end is formed at the connection end portion in such a fashion as to be bent in a "Z" shape upwardly from a distal end of the upper end spring positioned outwardly from an uppermost winding of the body spring, wherein a second contact-preventing/rigidity-reinforcing end is formed at the uppermost winding of the body spring positioned below the first contact-preventing/rigidity-reinforcing end in such a fashion as to be bent in a "Z" shape upwardly from a terminating point of the uppermost winding of the body spring to a starting point of the upper end spring, and wherein the exposure wire spring is further formed integrally with the lower end spring in such a fashion as to be disposed below the lower end spring, the first contact-preventing/rigidity-reinforcing end is further formed at a connection end portion extending between the lower end spring and the exposure wire spring, and the second contact-preventing/rigidity-reinforcing end is further formed at a connection portion extending between the lower end spring and the lowermost winding of the body spring.

According to another aspect of the present invention, there is provided a spring structure having a contact-preventing and rigidity-reinforcing function for a bed mattress which comprises a body spring formed in a coil shape, upper and lower end springs wound and extending horizontally at upper and lower portions of the body spring, an exposure wire spring formed integrally with the upper end spring in such a fashion as to be disposed above the upper end spring, and a connection end portion for integrally connecting the upper end spring and the exposure wire spring to each other, wherein a first contact-preventing/rigidity-reinforcing end is formed at the connection end portion in such a fashion as to be bent in a "Z" shape upwardly from a distal end of the upper end spring positioned outwardly from an uppermost winding of the body spring, wherein a second contact-preventing/rigidity-reinforcing end is formed at the uppermost winding of the body spring positioned below the first contact-preventing/rigidity-reinforcing end in such a fashion as to be bent in a "Z" shape upwardly from a terminating point of the uppermost winding of the body spring to a starting point of the upper end spring, and wherein the second contact-preventing/rigidity-reinforcing end is further formed at a connection portion extending between the lower end spring and the lowermost winding of the body spring.

Preferably, each of the first and second contact-preventing ends may have a height of 5 to 60mm.

Also preferably, the "Z"-shape bent angle of the first and second contact-preventing/rigidity-reinforcing ends with respect to a vertical axis of the body spring may be in the range between 0° and 70°.

### BRIEF DESCRIPTION OF THE DRAWINGS

The above and other objects, features and advantages of the present invention will be apparent from the following detailed description of the preferred embodiments of the invention in conjunction with the accompanying drawings, in which:
FIGs. 1a, 1b and 1c are a perspective view, a front view, and a side view illustrating a spring for a bed mattress according to the first embodiment of the present invention;
FIGs. 2a, 2b and 2c are a perspective view, a front view, and a side view illustrating the states where the spring according to the first embodiment of the present invention is assembled in a spring assembly;
FIGs. 3a and 3b are a front view and a side view illustrating the compression actions of the spring according to the first embodiment of the present invention;
FIGs. 4a, 4b and 4c are a perspective view, a front view, and a side view illustrating a spring for a bed mattress according to the second embodiment of the present invention;
FIG. 4d is a side view illustrating a state where a second contact-preventing/rigidity-reinforcing end is further formed at a lower end spring, which is similar to FIG. 4c;
FIGs. 5a, 5b and 5c are a perspective view, a front view, and a side view illustrating the states where the spring according to the second embodiment of the present invention is assembled in a spring assembly;
FIGs. 6a and 6b are a front view and a side view illustrating the compression actions of the spring according to the second embodiment of the present invention;
FIG. 7 is a side view illustrating a spring for a bed mattress according to the third embodiment of the present invention, in which an exposure wire spring is connected to a lower end spring thereof and which is identical to the spring according to the first embodiment of the present invention;
FIG. 8 is a side view illustrating a spring for a bed mattress according to the fourth embodiment of the present invention, in which an exposure wire spring is connected to a lower end spring thereof and which is identical to the spring according to the second embodiment of the present invention;
FIGs. 9a and 9b are perspective views illustrating a spring for a bed mattress according to the fifth embodiment of the present invention, in which an exposure wire spring is connected to an upper end spring thereof, wherein FIG. 9a shows a state where the exposure wire spring is formed at an upper portion thereof and FIG. 9b shows a state where the exposure wire spring is formed at upper and lower portions thereof;
FIGs. 10a and 10b are a perspective view and a side view illustrating a conventional spring structure according to the prior art;
FIGs. 11a, 11b and 11c are a perspective view, a front view and a side view illustrating a conventional spring structure having a dual buffer function according to the prior art;
FIG. 12 is a perspective view illustrating the conventional spring structure viewed from different angles for the sake of explanation of a disadvantage of the spring shown in FIGs. 11a and 11b; and
FIG. 13 is a cross-section view illustrating the structure of a conventional bed mattress.

### DETAILED DESCRIPTION OF THE PREFERRED EMBODIMENT

Reference will now be made in detail to the preferred embodiment of the present invention with reference to the attached drawings.

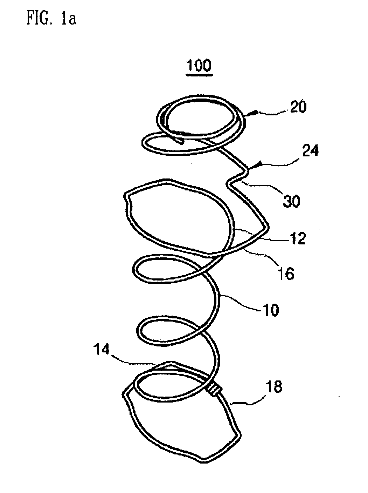

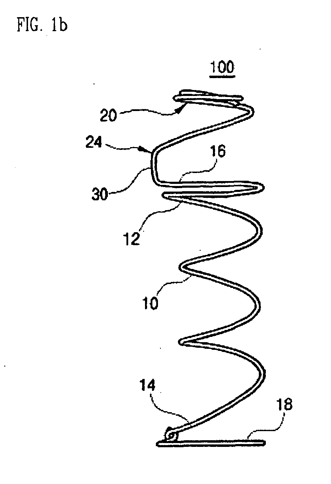

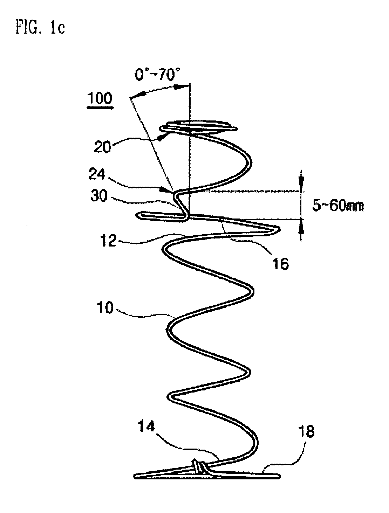

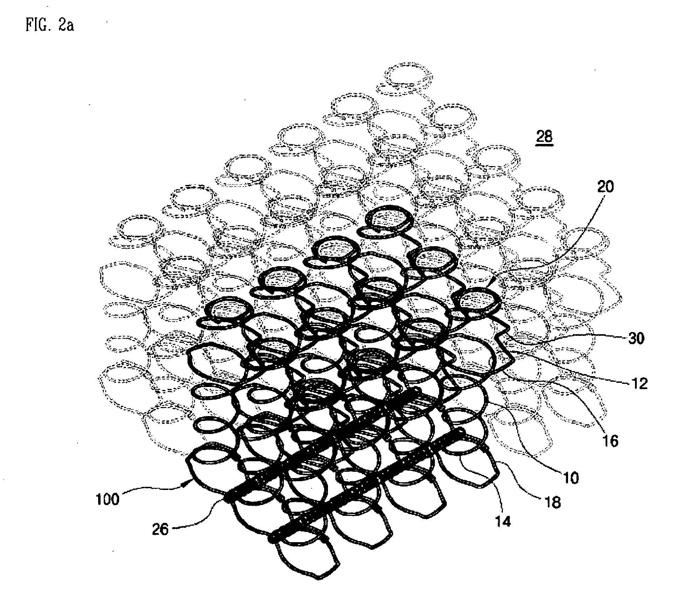

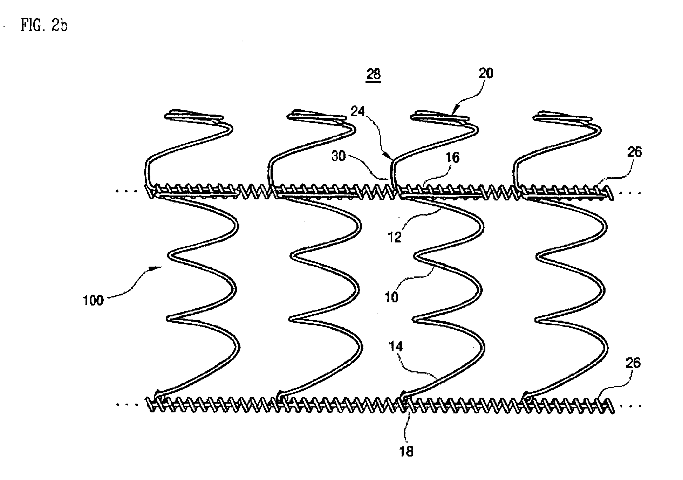

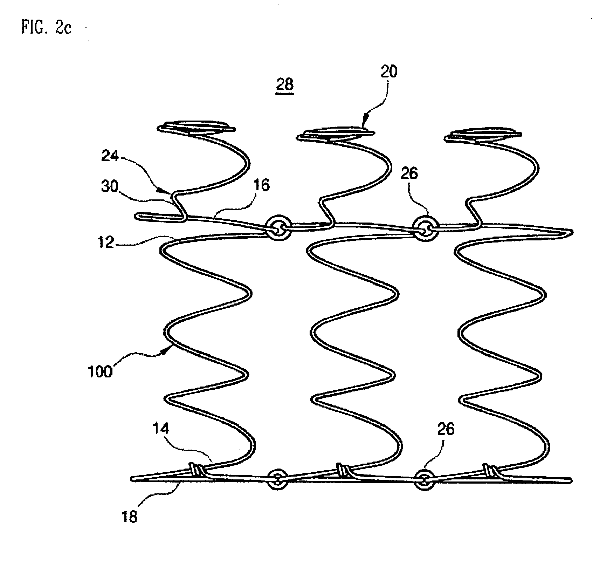

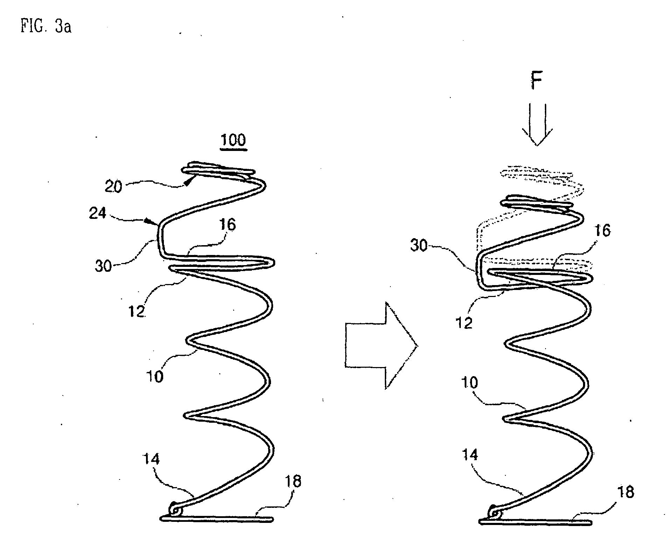

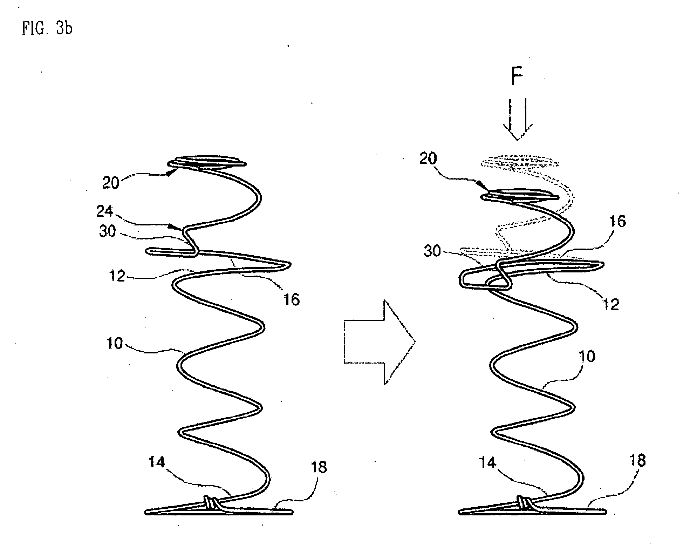

FIGs. 1a, 1b and 1c are a perspective view, a front view, and a side view illustrating a spring for a bed mattress according to the first embodiment of the present invention, FIGS. 2a, 2b and 2c are a perspective view, a front view, and a side view illustrating the states where the spring according to the first embodiment of the present invention is assembled in a spring assembly, and FIGs. 3a and 3b are a front view and a side view illustrating the compression actions of the spring according to the first embodiment of the present invention.

Referring to the drawings, a spring structure for a bed mattress, which performs a buffering function thereof separately depending on the magnitude of a load exerted to a bed mattress, comprises a body spring 10 formed in a coil shape, upper and lower end springs 16 and 18 wound and extending horizontally at upper and lower portions of the body spring, an exposure wire spring 20 formed integrally with the upper end spring 16 in such a fashion as to be disposed above the upper end spring 16, and a connection end portion 24 for integrally connecting the upper end spring and the exposure wire spring to each other.

Especially, the exposure wire spring 20 has a diameter smaller than that of the body spring 10 in such a fashion that the diameter thereof is smaller as it goes toward the top and is configured to be wound in a coil shape.

A single spring unit for the bed mattress including the body spring 10 and the exposure wire spring 20 is fabricated as a spring assembly 2, as shown in FIGs. 2a to 2c, which is composed of springs arranged spaced apart from one another at regular intervals along row and column directions over the entire area of the bed mattress, and helical coils 26 for securely engaging one side ends of the upper and lower end springs 16 and 18 of adjacent springs for bed mattress with one another.

Here, the spring 100 according to the first embodiment of the present invention features that a first contact-preventing/rigidity-reinforcing end 30 is formed at the connection end portion for integrally connecting the upper end spring 16 and the exposure wire spring 20 in such a fashion as to be bent in a "Z" shape upwardly from a distal end of the upper end spring positioned outwardly from an uppermost winding of the body spring 10.

More specifically, the first contact-preventing/rigidity-reinforcing end 30 is a wire section bent in a "Z" shape upwardly (vertically) from a distal end of the upper end spring 16 positioned outwardly from an uppermost winding 12 of the body spring 10.

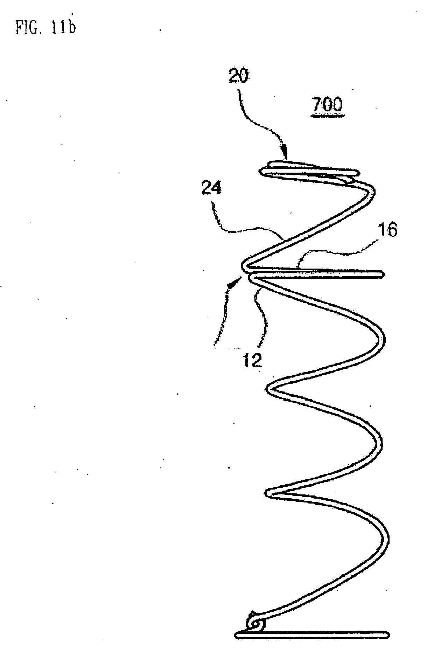

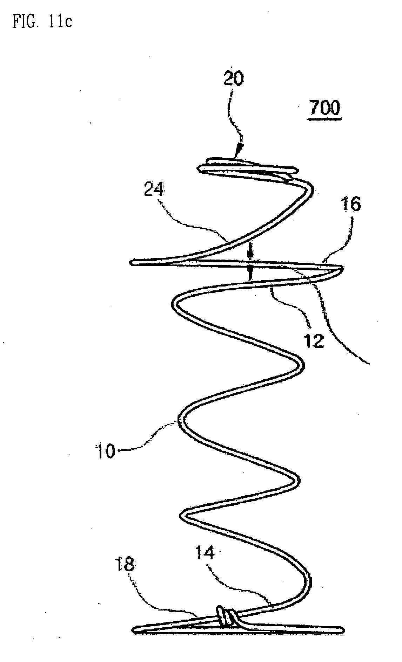

In other words, when viewed from the top, the first contact-preventing/rigidity-reinforcing end 30 is disposed outwardly from an uppermost winding 12 of the body spring 10 without being crossed or overlapped. Thus, upon the compression of the exposure wire spring 20 the first contact-preventing/rigidity-reinforcing end 30 descends while passing by the uppermost winding 12 of the body spring 10 without touching the uppermost winding 12, thereby preventing the contact between the connection end portion 24 and the uppermost winding 12 and a noise due to the contact occurring in the conventional spring having a dual buffer structure (see FIGs. 11a, 11b, 11c and 12).

However, in the spring structure according to the first embodiment of the present invention, as shown in FIGs. 3a and 3b, the first contact-preventing/rigidity-reinforcing end 30 is formed at the connection end portion 24. Thus, when a load is exerted to the bed mattress to compress the exposure wire spring 20, the first contact-preventing/rigidity-reinforcing end 30 descends while passing by the uppermost winding 12 of the body spring 10 positioned just therebelow without touching the uppermost winding 12, thereby fully eliminating the noise itself due to the contact between the connection end portion 24 of the exposure wire spring 20 and the uppermost winding 12.

Particularly, the first contact-preventing/rigidity reinforcing end 30 is enhanced in its rigidity while being bent in a "Z" shape, i.e., is enhanced in its rigidity required to support the exposure wire spring 20 so that a lateral displacement is not generated in which the exposure wire spring 20 is leaned to one side along with exertion of a load to the exposure wire spring 20 but stably ascends/descends vertically only so as to provide a uniform cushion sense.

More specifically, as the first contact-preventing/rigidity-reinforcing end 30 is enhanced in its rigidity while being bent in a "Z" shape, it is prevented from being displaced or leaned laterally and serves to stably support the exposure wire spring 20 disposed above the first contact-preventing/rigidity-reinforcing end 30. As a result, when the exposure wire spring 20 is applied with a load, it stably descends/ascends vertically only, but is not displaced laterally so as to provide a uniform cushion sense.

A spring structure for a bed mattress according to the second embodiment of the present invention will now be described hereinafter.

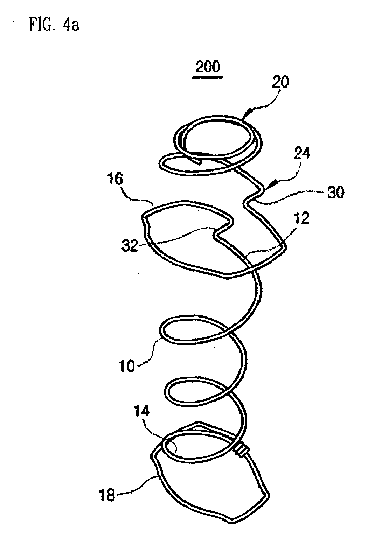

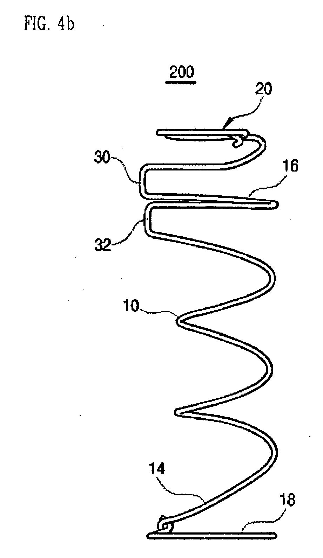

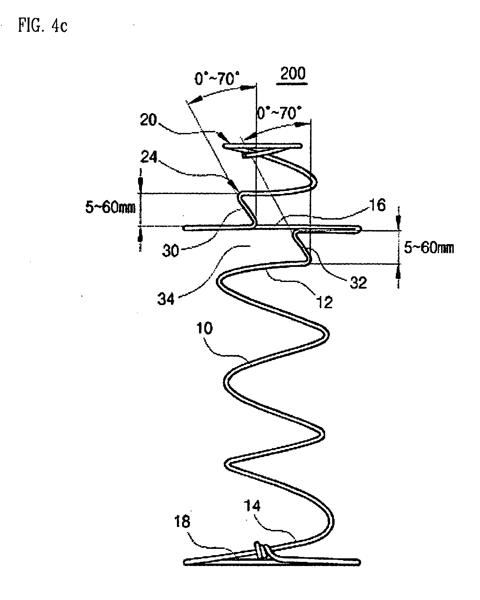

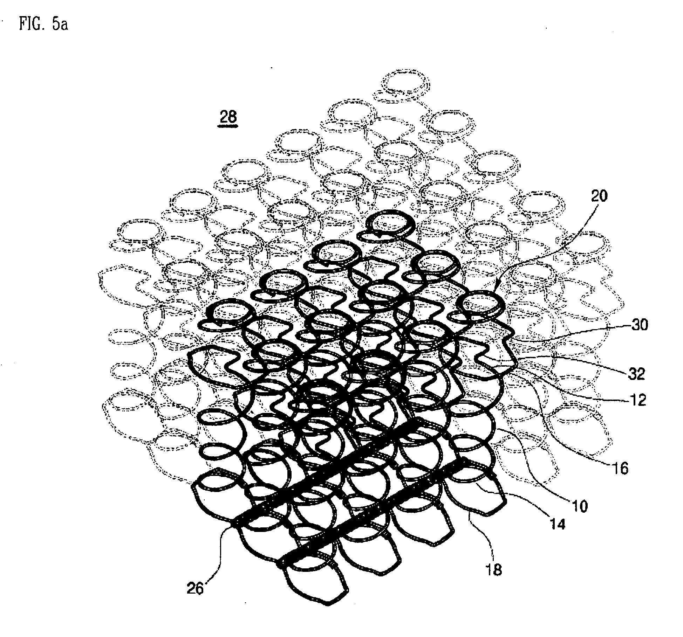

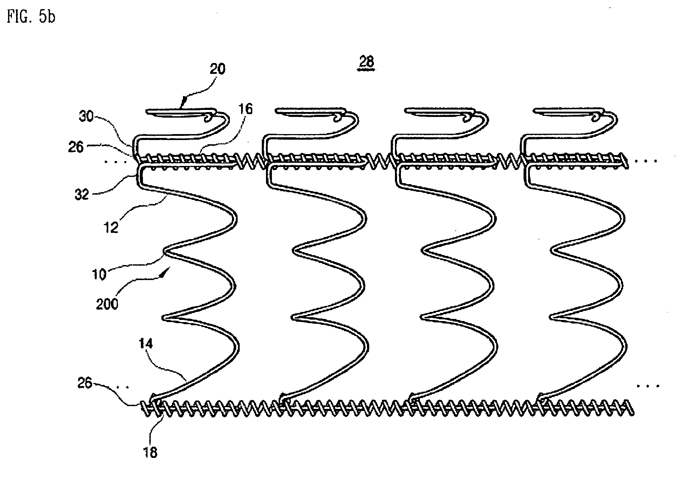

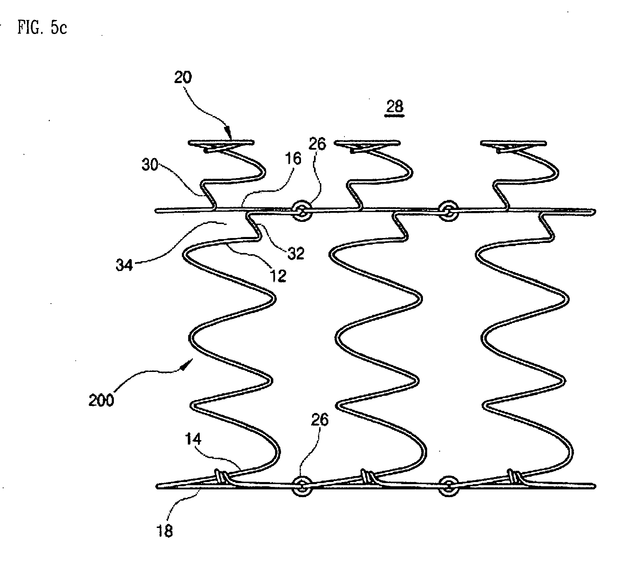

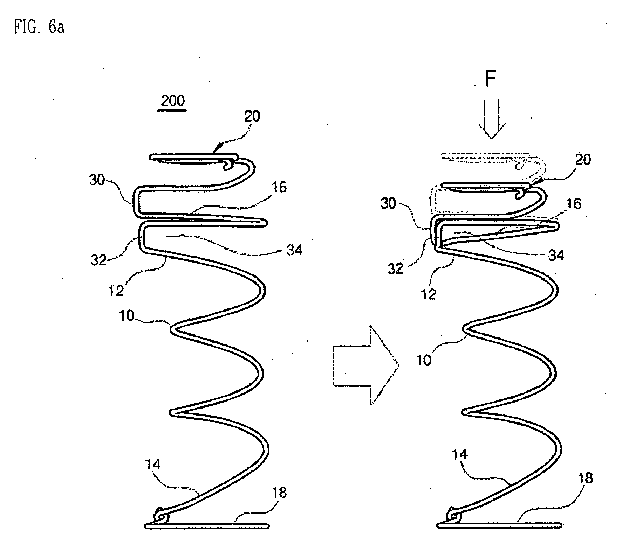

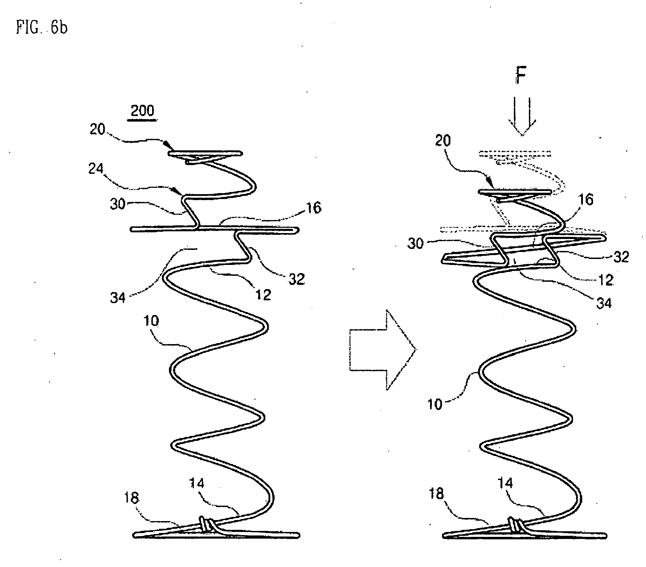

FIGs. 4a, 4b and 4c are a perspective view, a front view, and a side view illustrating a spring for a bed mattress according to the second embodiment of the present invention, FIGs. 5a, 5b and 5c are a perspective view, a front view, and a side view illustrating the states where the spring according to the second embodiment of the present invention is assembled in a spring assembly, and FIGs. 6a and 6b are a front view and a side view illustrating the compression actions of the spring according to the second embodiment of the present invention.

The spring 200 according to the second embodiment of the present invention, whose structure is identical to that of the spring 100 according to the first embodiment, features that a second contact-preventing/rigidity-reinforcing end 32 is formed at an uppermost winding 12 of the body spring 10 in such a fashion as to be bent in a "Z" shape.

More specifically, the second contact-preventing/rigidity-reinforcing end 32 is formed at a connection section between a terminating point of the uppermost winding 12 of the body spring 10 and a starting point of the upper end spring 16 in such a fashion as to be bent in a "Z" shape upwardly from the terminating point of the uppermost winding 12 to the starting point of the upper end spring 16.

At this time, as shown in FIG. 4b, a space defined behind the second contact-preventing/rigidity-reinforcing end 32, i.e., a space defined just above the uppermost winding of the body spring) is used as a contact-preventing space 34. The reason why the contact-preventing space 34 is formed is that when a load is applied to the bed mattress and simultaneously the exposure wire spring 20 is compressed, the first contact-preventing/rigidity-reinforcing end 30 is located at the space defined behind the second contact-preventing/rigidity-reinforcing end 32 while descending so that it does not come into close contact with an uppermost winding 12 of the body spring 10.

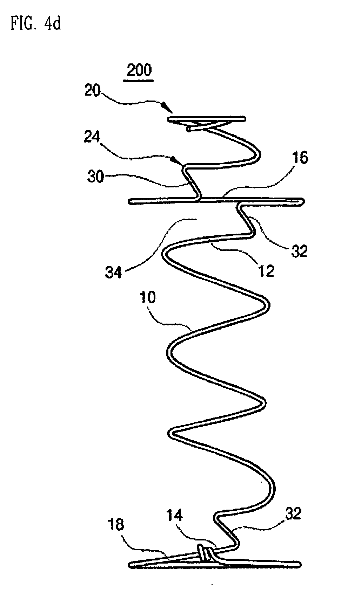

In the meantime, as shown in FIG. 4d, the second contact-preventing/rigidity-reinforcing end 32 is also formed at the lowermost winding 14 of the body spring 10 to further reinforce the support and resilient strength of the body spring 10.

That is, as shown in FIGs. 4d, 6a and 6b, when a load is exerted to the bed mattress to compress the exposure wire spring 20, the first contact-preventing/rigidity-reinforcing end 30 descends and is located at the space defined behind the second contact-preventing end 32, i.e., a contact-preventing space 34 so that the first contact-preventing/rigidity-reinforcing end 30 and the uppermost winding 12 of the body spring 10 do not come into close contact with each other, thereby fundamentally preventing generation of the noise due to the contact between the connection end portion 24 and the uppermost winding 12. And simultaneously, when the first contact-preventing/rigidity-reinforcing end 30 descends, the second contact-preventing/rigidity-reinforcing end 32 formed at the lowermost winding 14 of the body spring 10 acts to support the body spring 10 so as to further reinforce the resilient strength of the body spring 10.

In addition, the exposure wire spring 20 is compressed, the first and second contact-preventing/rigidity-reinforcing ends 30 and 32 are enhanced in its rigidity while being bent in a "Z" shape, i.e., is enhanced in its rigidity required to support the exposure wire spring 20 so that a lateral displacement is not generated in which the exposure wire spring 20 is leaned to one side along with exertion of a load to the exposure wire spring 20 but stably ascends/descends vertically only so as to provide a uniform cushion sense.

As such, in the spring structure according to the first and second embodiments of the present invention, the contact-preventing function of the first and second contact-preventing/rigidity-reinforcing ends 30 and 32 cause generation of the noise to be prevented completely and the enhanced rigidity prevents the lateral displacement of the exposure wire spring 20.

In the meantime, the height of the first and second contact-preventing/rigidity-reinforcing ends 30 and 32 is in the range between 5 to 60mm. The reason of limiting the height is that if the height thereof is less than 5mm, the formation itself of the first and second contact-preventing/rigidity-reinforcing ends 30 and 32 are difficult, and if the height thereof is more than 60mm, the intrinsic property of the spring is lost.

In addition, the inclination angle of the first and second contact-preventing/rigidity-reinforcing ends 30 and 32 with respect to a vertical axis of the body spring 10 is limited to the range between 0° and 70°. The reason of limiting the inclination angle is that if the inclination angle is 0°, the rigidity of the exposure wire spring 20 is the most favorable, and if the inclination angle is more than 70°, the rigidity of the exposure wire spring 20 becomes weak and the impact-absorbing capacity of the exposure wire spring 20 is deteriorated.

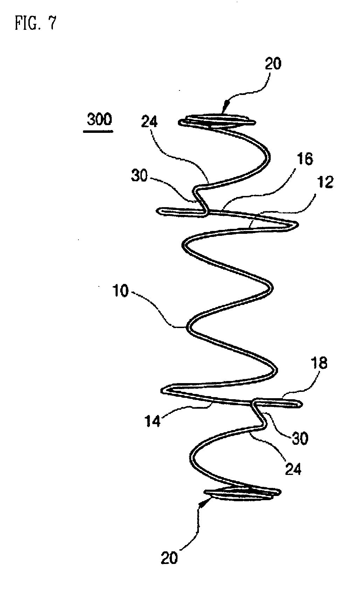

FIG. 7 is a side view illustrating a spring for a bed mattress according to the third embodiment of the present invention, in which an exposure wire spring is connected to a lower end spring thereof and which is identical to the spring according to the first embodiment of the present invention.

The spring 300 according to the third embodiment shown in FIG. 7 is characterized in that the exposure wire spring 20 is connected integrally with the upper end spring 16 in such a fashion as to be disposed above the upper end spring 16, and is connected integrally with the lower end spring 18 in such a fashion as to be disposed below the lower end spring 18. Of course, the first contact-preventing/rigidity-reinforcing end 30 is also formed between the lower end spring 18 and the exposure wire spring 20 for the purpose of prevention of the noise due to any contact therebetween and reinforcement of rigidity.

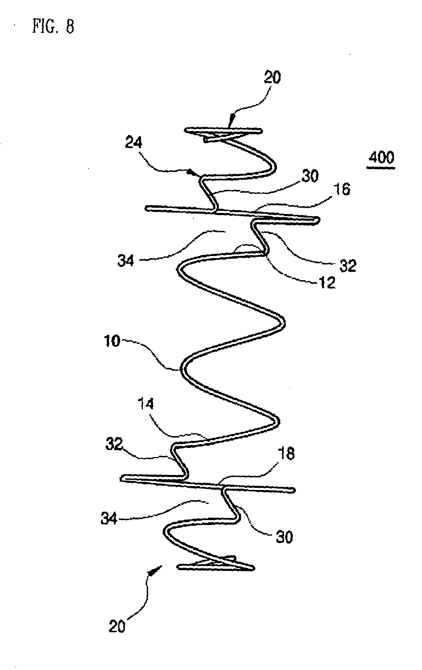

FIG. 8 is a side view illustrating a spring for a bed mattress according to the fourth embodiment of the present invention, in which an exposure wire spring is connected to a lower end spring thereof and which is identical to the spring according to the second embodiment of the present invention.

The spring 400 according to the fourth embodiment shown in FIG. 8 is characterized in that the exposure wire spring 20 is connected integrally with the upper end spring 16 in such a fashion as to be disposed above the upper end spring 16, and is connected integrally with the lower end spring 18 in such a fashion as to be disposed below the lower end spring 18. Of course, the first contact-preventing/rigidity-reinforcing end 30 is also formed between the lower end spring 18 and the exposure wire spring 20, and the second contact-preventing/rigidity-reinforcing end 32 is also formed between the lower end spring 18 and the lowermost winding 14 of the body spring 10.

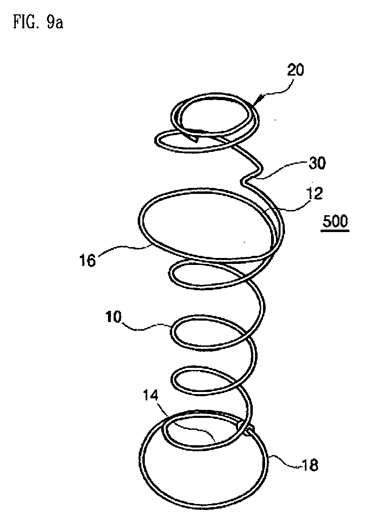

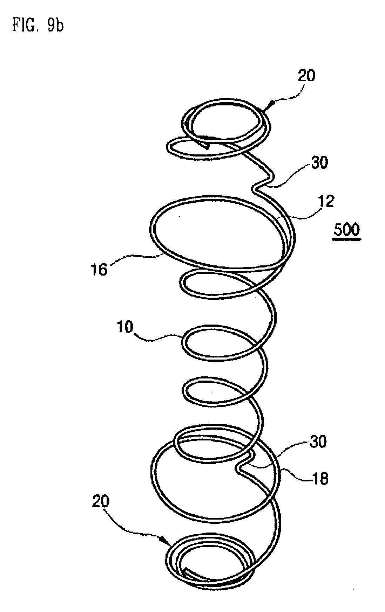

FIGs. 9a and 9b are perspective views illustrating a spring for a bed mattress according to the fifth embodiment of the present invention, in which an exposure wire spring is connected to an upper end spring thereof.

The spring 500 according to the fifth embodiment shown in FIGs. 9a and 9b is characterized in that the exposure wire spring 20 is connected integrally with the upper end spring 16 of a conventional coil shaped spring (see FIGs. 10a and 10b) in such a fashion as to be disposed above the upper end spring 16, or the upper end spring 16 and the lower end spring 18 in such a fashion as to be disposed above the upper end spring 16 and below the lower end spring 18. Of course, the first contact-preventing/rigidity-reinforcing end 30 is also formed between the upper end spring 16 and the exposure wire spring 20 or the upper end spring 16 and/or the lower end spring 18 and the exposure wire spring 20.

As such, according to the present invention, the exposure wire spring 20 may be connected to the upper end spring of the body spring 10, or the upper end spring and the lower end spring of the body spring 10 irrespective of the kind of the spring. In case of such connection of the exposure wire spring 20, the first contact-preventing/rigidity-reinforcing end 30 alone or the first and second contact-preventing/rigidity-reinforcing ends 30 and 32 are formed to prevent the contact between the springs and a noise due to the contact. Further, the exposure wire spring 20 may be configured in such a fashion as to ascend/descend vertically only without the lateral displacement thereof through the reinforcement of support rigidity of the exposure wire spring 20.

The spring according to the respective embodiments of the present invention as described above is a spring having a dual buffer structure fabricated by any one of the following steps of:
i) forming the first contact-preventing/rigidity-reinforcing end 30 in a "Z" shape at a connection portion extending between the upper end spring 16 and the exposure wire spring 20 positioned above the upper end spring 16,
ii) forming the first contact-preventing/rigidity-reinforcing end 30 in a "Z" shape at a connection portion extending between the upper end spring 16 and the exposure wire spring 20 positioned above the upper end spring 16, and simultaneously forming the second contact-preventing/rigidity-reinforcing end 32 in a "Z" shape at a portion extending from the uppermost winding 12 of the body spring 10 to the upper end spring 16,
iii) forming the first contact-preventing/rigidity-reinforcing end 30 in a "Z" shape at a connection portion extending between the upper end spring 16 and the exposure wire spring 20 positioned above the upper end spring 16, and simultaneously further forming the first contact-preventing/rigidity-reinforcing end 30 in a "Z" shape at a connection portion extending between the lower end spring 18 and the exposure wire spring 20 positioned below the lower end spring 18,
iv) forming the first contact-preventing/rigidity-reinforcing end 30 in a "Z" shape at a connection portion extending between the upper end spring 16 and the exposure wire spring 20 positioned above the upper end spring 16 and simultaneously further forming the second contact-preventing end 32 in a "Z" shape at a portion extending from the uppermost winding 12 of the body spring 10 to the upper end spring 16, as well as further forming the first contact-preventing/rigidity-reinforcing end 30 in a "Z" shape at a connection portion extending between the lower end spring 18 and the exposure wire spring 20 positioned below the lower end spring 18 and simultaneously further forming the second contact-preventing/rigidity-reinforcing end 32 in a "Z" shape at a portion extending from the lower end spring 18 to the lowermost winding 14 of the body spring 10, and
v) forming the first contact-preventing/rigidity-reinforcing end 30 in a "Z" shape at a connection portion extending between the upper end spring 16 and the exposure wire spring 20 positioned above the upper end spring 16 and simultaneously further forming the second contact-preventing/rigidity-reinforcing end 32 in a "Z" shape at a portion extending from the uppermost winding 12 of the body spring 10 to the upper end spring 16, as well as further forming the second contact-preventing/rigidity-reinforcing end 32 at a portion extending from the lower end spring 18 to the lowermost winding 14 of the body spring 10.

In the spring having the dual buffer structure, the body spring and the exposure wire spring perform a buffering function thereof separately depending on the magnitude of the load exerted to the bed mattress to thereby provide a stable and uniform cushion sense.

Particularly, the first contact preventing/rigidity-reinforcing end 30, or the first and second contact-preventing/rigidity-reinforcing ends 30 and 32 concurrently achieves a contact-preventing function in which the connection end portion is not in close contact with an uppermost winding and/or a lowermost winding of the body spring as well as a rigidity-reinforcing function in which the exposure wire spring is guided to stably ascend/descend vertically only, but not displaced laterally, when a load is exerted to the exposure wire spring, to thereby provide advantages of preventing the deformation of the spring and prolonging the lifespan of the spring.

As apparent from the foregoing, according to the inventive spring structure having a contact-preventing and rigidity-reinforcing function for a bed mattress, the structure of a connection end portion for connecting a body spring and an exposure wire spring to each other is improved such that the connection end portion is not in close contact with an uppermost winding of the body spring and the rigidity required to support the exposure wire spring is enhanced, so that the following merits are provided:
a) The first contact-preventing/rigidity-reinforcing end is formed at a connection end portion extending between the body spring and the exposure wire spring, so that when the exposure wire spring is compressed by means of a load exerted to the bed mattress, the first contact-preventing/rigidity-reinforcing end does not bring into contact with the uppermost winding of the body spring while descending, thereby easily preventing a deformation of the spring due to a frictional contact along with the fundamental prevention of a contact noise as well as preventing the lateral displacement of the exposure wire spring while guiding only the vertical movement of the exposure wire spring.
b) The second contact-preventing/rigidity-reinforcing end is further formed between the upper end spring and the body spring in addition to the first contact-preventing/rigidity-reinforcing end, so that the first contact-preventing/rigidity-reinforcing end is positioned at the space defined behind the second contact-preventing/rigidity-reinforcing end while descending or passing by the uppermost winding of the body spring without touching the uppermost winding although it descends, thereby further easily preventing a deformation of the spring due to a frictional contact along with the fundamental prevention of a contact noise, as well as providing a structure having a greater rigidity for preventing the lateral displacement of the exposure wire spring.
c) Resultantly, the present invention concurrently achieves a contact-preventing function in which the connection end portion is not in close contact with an uppermost winding and/or a lowermost winding of the body spring as well as a rigidity-reinforcing function in which the exposure wire spring is guided to stably ascend/descend vertically only, but not displaced laterally, when a load is exerted to the exposure wire spring, to thereby provide advantages of preventing the deformation of the spring and prolonging the lifespan of the spring.

While the present invention has been described with reference to the particular illustrative embodiments, it is not to be restricted by the embodiments but only by the appended claims. It is to be appreciated that those skilled in the art can change or modify the embodiments without departing from the scope thus defined.

## Claims

1. A spring structure having a contact-preventing and rigidity-reinforcing function for a bed mattress which comprises a body spring (10) formed in a coil shape, upper and lower end springs (16, 18) wound and extending horizontally at upper and lower portions of the body spring (10),
**characterized by**
an exposure wire spring (20) which is formed integrally with the upper end spring (16) in such a fashion as to be disposed above the upper end spring (16), and a connection end portion (24) for integrally connecting the upper end spring (16) and the exposure wire spring (20) to each other,
wherein a first contact-preventing/rigidity-reinforcing end (30) is formed at the connection end portion (24) in such a fashion as to be bent in a "Z" shape upwardly from a distal end of the upper end spring (16) positioned outwardly from an uppermost winding (12) of the body spring.

2. The spring structure according to claim 1, wherein a second contact-preventing/rigidity-reinforcing end (30) is formed at the uppermost winding (12) of the body spring (10) positioned below the first contact-preventing/rigidity-reinforcing end (30) in such a fashion as to be bent in a "Z" shape upwardly from a terminating point of the uppermost winding (12) of the body spring (10) to a starting point of the upper end spring (16).

3. The spring structure according to claim 1, wherein the exposure wire spring (20) is further formed integrally with the lower end spring (18) in such a fashion as to be disposed below the lower end spring, and the first contact-preventing/rigidity-reinforcing end (30) is further formed at a connection end portion (24) extending between the lower end spring (18) and the exposure wire spring.

4. The spring structure according to claim 2, wherein the exposure wire spring (20) is further formed integrally with the lower end spring (18) in such a fashion as to be disposed below the lower end spring, the first contact-preventing/rigidity-reinforcing end (30) is further formed at a connection end portion (24) extending between the lower end spring (18) and the exposure wire spring, and the second contact-preventing/rigidity-reinforcing end (32) is further formed at a connection portion extending between the lower end spring (18) and the lowermost winding (14) of the body spring (10).

5. The spring structure according to claim 2, wherein the second contact-preventing/rigidity-reinforcing end (32) is further formed at a connection portion extending between the lower end spring (18) and the lowermost winding (14) of the body spring (10).

6. The spring structure as set forth in any one of claims 1 to 5, wherein each of the first and second contact-preventing ends (30, 32) may have a height of 5 to 60mm.

7. The spring structure as set forth in any one of claims 1 to 5, wherein the "Z"-shape bent angle of the first and second contact-preventing/rigidity-reinforcing ends (30, 32) with respect to a vertical axis of the body spring (10) may be in the range between 0° and 70°.

## Patentansprüche

1. Federstruktur mit einer kontaktvermeidenden und steifikeitsverstärkenden Funktion für eine Bettmatratze, wobei die Federstruktur einen Federkörper (10), der schraubenförmig ausgebildet ist, obere und untere Endfedern (16, 18), die gewickelt sind und sich horizontal an oberen und unteren Abschnitten des Federkörpers (10) erstrecken, aufweist,
**gekennzeichnet durch**
eine Aufnahmedrahtfeder (20), die integral mit der oberen Endfeder (16) in solch einer Weise ausgebildet ist, dass sie über der oberen Endfeder (16) angeordnet ist, und einen Verbindungsendabschnitt (24) zum integralen miteinander Verbinden der oberen Endfeder (16) und der Aufnahmedrahtfeder (20),
wobei ein erstes kontaktvermeidendes / steifigkeitsverstärkendes Ende (30) an dem Verbindungsendabschnitt (24) in solch einer Weise ausgebildet ist, dass es "Z"-förmig abgewinkelt aufwärts neigend von einem distalen Ende der oberen Endfeder (16) und nach außen von der obersten Windung (12) des Federkörpers positioniert ist.

2. Federstruktur nach Anspruch 1,
wobei ein zweites kontaktvermeidendes / steifigkeitsverstärkendes Ende (32) an der obersten Windung (12) des Federkörpers (10) ausgebildet ist und unterhalb des ersten kontaktvermeidenden / steifigkeitsverstärkenden Endes (30) in solch einer Weise positioniert ist, dass es von einem Endpunkt der obersten Windung (12) des Federkörpers (10) zu einem Startpunkt der oberen Endfeder (16) "Z"-förmig abgewinkelt aufwärts neigend positioniert ist.

3. Federstruktur nach Anspruch 1,
wobei die Aufnahmedrahtfeder (20) ferner integral mit der unteren Endfeder (18) in solch einer Weise ausgebildet ist, dass sie unterhalb der unteren Endfeder angeordnet ist und das erste kontaktvermeidende / steifigkeitsverstärkende Ende (30) ferner an einem Verbindungsendabschnitt (24), der sich zwischen der unteren Endfeder (18) und der Aufnahmedrahtfeder erstreckt, ausgebildet ist.

4. Federstruktur nach Anspruch 2,
wobei die Aufnahmedrahtfeder (20) ferner integral mit der unteren Endfeder (18) in solch einer Weise ausgebildet ist, dass sie unterhalb der unteren Endfeder angeordnet ist, wobei das erste kontaktvermeidende / steifigkeitsverstärkende Ende (30) ferner an einem Verbindungsendabschnitt (24), der sich zwischen der unteren Endfeder (18) und der Aufnahmedrahtfeder erstreckt, ausgebildet ist und das zweite kontaktvermeidende / steifigkeitsverstärkende Ende (32) ferner an einem Verbindungsabschnitt, der sich zwischen der unteren Endfeder (18) und der untersten Windung (14) des Federkörpers (10) erstreckt, ausgebildet ist.

5. Federstruktur nach Anspruch 2,
wobei das zweite kontaktvermeidende / steifigkeitsverstärkende Ende (32) ferner an einem Verbindungsabschnitt, der sich zwischen der unteren Endfeder (18) und der untersten Windung (14) des Federkörpers (10) erstreckt, ausgebildet ist.

6. Federstruktur nach irgendeinem der dargelegten Ansprüche 1 bis 5,
wobei jedes von den ersten und zweiten kontaktvermeidenden Enden (30, 32) eine Höhe von 5 bis 60 mm aufweist.

7. Federstruktur nach irgendeinem der dargelegten Ansprüche 1 bis 5,
wobei der "Z"-förmige Neigungswinkel der ersten und zweiten kontaktvermeidenden / steifigkeitsverstärkenden Enden (30, 32) in Bezug auf die vertikale Achse des Federkörpers (10) im Bereich zwischen 0° und 70° ist.

## Revendications

1. Structure de ressort ayant une fonction empêchant le contact et renforçant la rigidité pour matelas de lit qui comprend un ressort formant corps (10) ayant une forme hélicoïdale, des ressorts d'extrémité supérieur et inférieur (16, 18) enroulés et s'étendant horizontalement au niveau de parties supérieure et inférieure du ressort formant corps (10),
**caractérisée par**
un ressort en fil métallique à nu (20) qui est formé d'un seul tenant avec le ressort d'extrémité supérieur (16) de façon à être disposé au-dessus du ressort d'extrémité supérieur (16), et une partie d'extrémité de connexion (24) pour relier d'un seul tenant le ressort d'extrémité supérieur (16) et le ressort en fil métallique à nu (20) l'un à l'autre,
dans laquelle une première extrémité empêchant le contact / renforçant la rigidité (30) est formée au niveau de la partie d'extrémité de connexion (24) de façon à être pliée en une forme de « Z » vers le haut à partir d'une extrémité distale du ressort d'extrémité supérieur (16) positionné à l'extérieur par rapport à un enroulement supérieur (12) du ressort formant corps.

2. Structure de ressort selon la revendication 1, dans laquelle une seconde extrémité empêchant le contact / renforçant la rigidité (30) est formée au niveau de l'enroulement supérieur (12) du ressort formant corps (10) positionné au-dessous de la première extrémité empêchant le contact / renforçant la rigidité (30) de façon à être pliée en une forme de « Z » vers le haut à partir d'un point de terminaison de l'enroulement supérieur (12) du ressort formant corps (10) jusqu'à un point de départ du ressort d'extrémité supérieur (16).

3. Structure de ressort selon la revendication 1, dans laquelle le ressort en fil métallique à nu (20) est en outre formé d'un seul tenant avec le ressort d'extrémité inférieur (18) de façon à être disposé au-dessous du ressort d'extrémité inférieur, et la première extrémité empêchant le contact / renforçant la rigidité (30) est en outre formée au niveau d'une partie d'extrémité de connexion (24) s'étendant entre le ressort d'extrémité inférieur (18) et le ressort en fil métallique à nu.

4. Structure de ressort selon la revendication 2, dans laquelle le ressort en fil métallique à nu (20) est en outre formé d'un seul tenant avec le ressort d'extrémité inférieur (18) de façon à être disposé au-dessous du ressort d'extrémité inférieur, la première extrémité empêchant le contact / renforçant la rigidité (30) est en outre formée au niveau d'une partie d'extrémité de connexion (24) s'étendant entre le ressort d'extrémité inférieur (18) et le ressort en fil métallique à nu, et la seconde extrémité empêchant le contact / renforçant la rigidité (32) est en outre formée au niveau d'une partie de connexion s'étendant entre le ressort d'extrémité inférieur (18) et l'enroulement inférieur (14) du ressort formant corps (10).

5. Structure de ressort selon la revendication 2, dans laquelle la seconde extrémité empêchant le contact / renforçant la rigidité (32) est en outre formée au niveau d'une partie de connexion s'étendant entre le ressort d'extrémité inférieur (18) et l'enroulement inférieur (14) du ressort formant corps (10).

6. Structure de ressort selon l'une quelconque des revendications 1 à 5, dans laquelle chacune des première et seconde extrémités empêchant le contact / renforçant la rigidité (30, 32) peut avoir une hauteur de 5 à 60 mm.

7. Structure de ressort selon l'une quelconque des revendications 1 à 5, dans laquelle l'angle de pliage en forme de « Z » des première et seconde extrémités empêchant le contact / renforçant la rigidité (30, 32) par rapport à un axe vertical du ressort formant corps (10) peut être dans la plage comprise entre 0° et 70°.
